# EUROPEAN PATENT SPECIFICATION

(11) **EP 1 228 025 B1**
(45) Date of publication and mention of the grant of the patent: **06.10.2004**
(21) Application number: 00957667.9
(22) Date of filing: 22.08.2000
(51) Int. Cl.: C07C 51/265, C07C 51/487, C07C 63/36

(54) **A METHOD FOR SOLVENT FREE HYDRODEBROMINATION IN THE PRODUCTION OF AROMATIC CARBOXYLIC ACIDS**
EIN VERFAHREN FÜR LÖSEMITTELFREIE HYDRODEBROMIERUNG IN DER HERSTELLUNG VON AROMATISCHEN CARBONSÄUREN
PROCEDE D'HYDRODEBROMURATION SANS SOLVANT DANS LA PRODUCTION D'ACIDES CARBOXYLIQUES AROMATIQUES

(30) Priority: 30.08.1999 US 151607 P
(43) Date of publication of application: 07.08.2002
(60) Divisional application: 04018340.2
(73) Proprietor: Mossi & Ghisolfi International S.A., 1528 Luxembourg (LU)
(72) Inventor: KREITMAN, Keith, M., Houston, TX 77095 (US); POTTER, Michael, W., Houston, TX 77478 (US)
(74) Representative: Gerbino, Angelo
(86) International application number: PCT/US2000/023016
(87) International publication number: WO 2001/016076

(56) References cited:
- US-A- 5 292 934

## Description

### CROSS REFERENCE

This application is related to U. S. Application Ser. Nos. 60/151,577, 60/151,498, 60/151,602, 60/151,603, 60/151,529, 60/151,489, 60/151,604, 60/151,606, 60/151,589, 60/151,497, 60/150,590 and 60/151,578

### FIELD OF THE INVENTION

This invention relates to the production of aromatic carboxylic acids by liquid phase oxidation using a bromine-promoted heavy metal catalyst. In particular it relates to hydrodebromination of the crude aromatic carboxylic acid product. Still more particularly, it relates to a method of hydrodebromination of crude product aromatic carboxylic acids which demonstrates several distinct advantages over any method currently available in the art. The present invention provides for the removal of acetic acid and oxidation catalyst species prior to hydrodebromination, operates efficiently at significantly lower temperatures than would be indicated by what is known in the art, and does not require a solvent.

### BACKGROUND OF THE INVENTION

Aromatic carboxylic acids are useful organic compounds. They can be used in the preparation of other organic compounds or as monomers for the preparation of polymeric materials.

Aromatic carboxylic acids are often prepared by the liquid phase, heavy metal catalyzed oxidation of alkyl or acyl substituted aromatic compounds to the corresponding aromatic carboxylic acid. For example, U. S. Pat. Nos. 2,833,816; 3,870,754; 4,933,491; and 4,950,786 disclose such oxidation methods. Such a reaction has drawbacks. Incomplete oxidation of a methyl group produces an aldehyde group instead of a carboxylic acid group. In addition, when a promoter such as bromine is used during liquid phase oxidation, brominated aromatic carboxylic acids are produced.

The reduction to acceptable levels of heavy metal catalyst and impurities remaining in the crude product after oxidation is a problem in the oxidation methods currently available in the art. Current methods result in product with an undesirable level of impurities trapped in the compound when the product is a solid or otherwise trapped in the crude reaction product where the crude product and reaction mixture is in liquid phase. Current methods can also result in the necessity for expensive metallurgy in any subsequent steps in the process.

U. S. 5,292,934 discloses a method for preparing an aromatic carboxylic acid by oxidizing an aromatic compound having at least one oxidizable alkyl or acyl group with oxygen in the presence of a low molecular weight carboxylic acid solvent and a heavy metal at a temperature of 121°C to 232°C (250°F to 450°F), heating the product to 288°C (550°F), and recovering the carboxylic acid. In this reference a procedure is employed to debrominate the resulting product at very high temperatures. High temperature hydrodebromination such as this can cause the unnecessary breakdown of desirable compounds.

There is a need in the art for an effective method of removing oxidation catalysts components, such as bromine, and other impurities, from crude product aromatic carboxylic acids prepared by liquid phase oxidation. It would be especially desirable if such a debromination method were effective without the use of extreme temperatures or solvents.

### SUMMARY

In accordance with the foregoing the present invention is an improved method for hydrodebromination of a crude product aromatic carboxylic acid prepared by liquid phase oxidation of an aromatic compound having at least one oxidizable alkyl or acyl group with an oxygen-containing gas, in a solvent comprising a low molecular weight carboxylic acid, in the presence of an oxidation catalyst comprising heavy metal components, and at a reaction temperature of 21°C to 232°C (250 to 450°F), thereby fonning an oxidation reaction product mixture.

Said hydrodebromination process comprising:
recovering said crude aromatic carboxylic acid product of oxidation by distinction;
extracting said crude aromatic monocarboxylic acid product with water, and
treating said crude aromatic carboxylic acid product with hydrogen over a catalyst selected from Group VIII of the Periodic Table, optionally on a support, at a temperature of from about 150-320°C and a hydrogen partial pressure of from 138 to 2,068 kPag (20 to 300 psig), thereby producing crude aromatic carboxylic acid product containing significantly reduced organic bromine compounds.

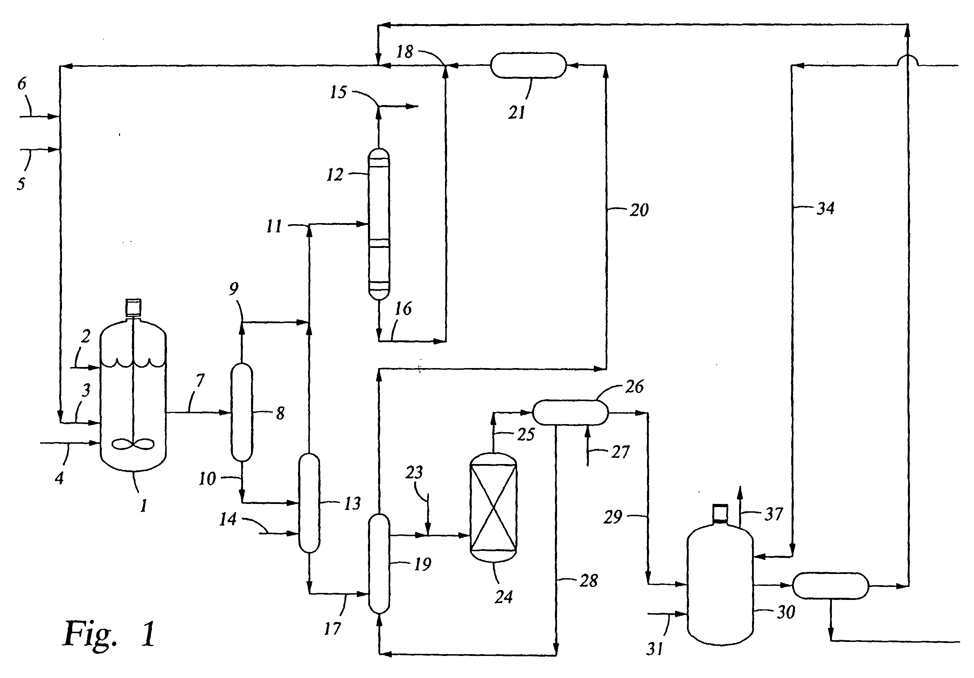

### BRIEF DESCRIPTION OF THE DRAWING

The Figure is a process flow diagram illustrating one embodiment of the hydrodebromination process.

### DETAILED DESCRIPTION OF THE INVENTION

The process of this invention greatly reduces levels of bromine in an oxidation product reaction mixture. The oxidation reaction product mixture used in the hydrodebromination method of this invention is obtained from the liquid phase, heavy metal catalyzed oxidation of an alkyl- or acyl substituted aromatic compound to the corresponding aromatic carboxylic acid. Particularly suitable aromatic feed components are those which result in a crude product mixture which, after removal of solvent, melts without substantial decomposition. Such compounds would typically have one or more aromatic rings having an oxidizable alkyl or acyl group wherein the alkyl or acyl groups contain from 1 to about 6 carbon atoms, inclusive. Preferably, the oxidizable alkyl or acyl group is methyl, ethyl, isopropyl, acetyl, or formyl. Examples of suitable aromatic feed compounds include: o-xylene, m-xylene, p-xylene. Examples of useful naphthalene-based feed compounds include: 1-methyl- and 2-methylnaphthalene, 1-ethyl-and 2-ethylnaphthalene, 1-isopropyl- and 2-isopropylnaphthalene.

The preferred embodiment and examples demonstrate the hydrodebromination of crude naphthoic acid prepared from an 85 to 90% methylnaphthalene stream. The present process for hydrodebromination of aromatic monocarboxylic acids is capable of reducing ppms of bromine remaining on said aromatic monocarboxylic acids from the heavy metal oxidation catalysts to levels below 50ppm, and even lower in some examples.

Methylnaphthalene streams used in this invention were prepared as described in U. S. Pat. Application Ser. No. 60/151,577 , filed of even date, and incorporated by reference herein in its entirety. These methylnaphthalene streams are substantially free of sulfur, but contain hydrocarbon impurities that cannot be readily separated from the methylnaphthalene. These remaining hydrocarbon impurities consist primarily of substituted indanes, which may comprise up to about 15% by weight of the total stream.

The methylnaphthalene stream is first oxidized with air to form a crude naphthoic acid stream, using acetic acid as a solvent, and a catalyst comprising cobalt, manganese and bromine. Any suitable oxidation process may be employed here. A particularly suitable process is described in U.S. 3,856,855, incorporated by reference herein in its entirety. In the oxidation reaction step, a methylnaphthalene stream is introduced into an oxidation reactor along with a solvent, catalyst, and an oxygen-containing gas. The methylnaphthalene stream is approximately 85% pure and contains a mixture of 1-methylnaphthalene and 2-methylnaphthalene, which are oxidized to form 1-naphthoic acid and 2-naphthoic acid, respectively.

Suitable solvents for the oxidation reaction include carboxylic acids. The preferred oxidation solvent is a low molecular weight monocarboxylic acid having 2 to 6 carbon atoms, with acetic acid being preferred.

The catalyst preferably comprises a bromine-containing compound and at least one of a cobalt- and manganese-containing compound. Preferably, the catalyst comprises cobalt-, manganese-, and bromine-containing components. Bromine containing components known in the art such as hydrogen bromide, molecular bromine and sodium bromide can be used. Additional catalyst components that are known in the art and may improve the rate or selectivity of the reaction include zirconium, hafnium, and cerium.

A source of molecular oxygen is also required, and typically it is air.

A suitable temperature range for the oxidation is from 100°C to 200°C. The preferred temperature range is from 130°C to 170°C, preferably 150°C. The oxidation step is normally run under refluxing conditions in order to remove the considerable heat evolved by the oxidation reactions, therefore the heat balance at the chosen temperature will determine the actual operating pressure.

Suitable pressures are in the range of 0 to 2,758 kPag (0 to 400 psig), preferably 689 to 2,068 kPag (100 to 300 psig). As noted in copending U.S. Pat. Application. Ser. No. 60/151,577, due to the fact that the steps following the oxidation allow for the efficient recycle of unreacted methylnaphthalene and intermediate oxidation products, the oxidation need not be carried out under conditions which favor high conversion. This allows lower reaction temperatures, lower concentrations of catalyst and acetic acid solvent, and shorter reaction times, leading to significantly reduced losses of acetic acid due to burning, a lighter colored product, and a significantly lower amount of bromine that is organically bound to the naphthoic acid product.

The oxidation can be performed either in a batch, continuous, or semicontinuous mode. In the present process the preferred operation is a continuous, integrated process, as described in copending U.S. Ser. No. 60/151,577 . Using the preferred methylnaphthalene feed in the oxidation step, methylnaphthalene is converted primarily to naphthoic acid, and water is formed as a by-product. A lesser portion of the methylnaphthalene remains unreacted, or is converted to naphthaldehyde, bromonaphthoic acid, and benzene polycarboxylic acids, such as phthalic and trimellitic acids. The substituted indanes that are present as impurities in the feed are also converted to similar benzene polycarboxylic acid. A number of other impurities and reaction by-products may be present depending on the oxidation reaction conditions used, the aromatic feed compound selected, the oxidation catalysts, and the levels of the catalyst selected.

In the present invention, the object is to remove bromine to extremely low levels.

According to the method of the present invention, after the oxidation step, the acetic acid solvent and produced water are removed from the crude naphthoic acid oxidation product via distillation, and steam stripping. Acetic acid is separated from the water by distillation in a second distillation column, and recycled to the oxidation reactor.

Water flows countercurrently through several steps in the process in which the naphthoic acid is washed. The first step using water is a water extraction. The recovered crude naphthoic acid product is extracted with water in an aqueous extraction reactor at a temperature of 140 to 160°C. The temperature should be above the melting point of the crude napthoic acid in the water. This water extraction step significantly reduces the amount of ionic bromine, by-product benzene polycarboxylic acids, and catalyst components in the crude naphthoic acid. These catalyst components are recovered from the water stream and recycled to the oxidation reactor.

In the hydrodebromination reaction step, crude molten naphthoic acid is treated with hydrogen over a catalyst to substantially reduce the amount of bromine that is organically-bound to the naphthoic acid products formed in the oxidation. The mixture is treated with hydrogen by passing the solvent-free stream over a carbon-supported palladium catalyst, converting the bromonaphthoic acid into naphthoic acid and ionic bromine. The ionic bromine created in this step is removed from the mixture by a second extraction with water. Benzene polycarboxylic acids, cobalt, and manganese are also removed from the remaining molten organic mixture by extraction with water and recycled to the oxidation step. This step minimizes the amount of bromine that is fed to the downstream sections of the process, where the presence of bromine would require more expensive metallurgy.

A suitable temperature for the debromination in this process is from about 150° to 320°C, preferably from 220° to 280°C. A suitable hydrogen partial pressure range is from 138 to 2,068 kPag (20 to 300 psig), preferably from 345 to 1,379 kPag (50 to 200 psig) and most preferably 689 kPag (100 psig). Excessive hydrogen partial pressure leads to partial saturation of the aromatic ring structures of naphthoic acid.

Suitable catalysts for the hydrodebromination include palladium, platinum, rhenium, ruthenium and alloys of these metals with others, for example palladium/silver or palladium/nickel. The catalyst is preferably on a support material selected from the group consisting of alumina, titania or carbon. Most preferably the catalyst is palladium supported on carbon.

When a Group VIII noble metal catalyst is used the noble metal is present in the catalyst typically in amount of 0.1 wt% to 5 wt% based on the total weight of the catalyst. A preferred catalyst is platinum, ruthenium or palladium on carbon wherein the metal is present in an amount of 0.01% to 1.0% based on the weight of the catalyst.

Other catalyst support materials could be employed, as long as they are stable under the reaction conditions.

Under the preferred conditions, this hydrodebromination step is capable of reducing the amount of bromine that is organically bound to naphthoic acid to very low levels.

There are a number of distinctions between the present invention and what is known about debromination from the art. An example of other work in the field relating to debromination is U. S. 5,292,934. That patent discloses a process suitable for the oxidation of a dialkyl-, an alkyl- acyl-or diacylnaphthalene compound to the corresponding naphthalene dicarboxylic acid. In '934 debromination of 2,6 naphthalene dicarboxylic acid is carried out thermally, by heating to a temperature as high as 343-371°C (650-700°F), in the presence of acetic acid solvent and a hydrogenation catalyst. A disadvantage of the requirement for high temperatures is that some desirable compounds are broken up. In contrast, the present invention operates well at significantly lower temperatures, does not require a solvent to carry out the hydrodebromination, and provides for the removal of acetic acid and oxidation catalyst species which are expected to have a negative effect on the hydrodebromination catalyst life.

Following hydrodebromination, the crude naphthoic acid is again extracted with water to remove ionic bromine created during the hydrodebromination reaction. The exiting water is routed to the previous water extraction step.

A critical difference between the present invention and what is typical in the art is that the crude oxidation product of the present invention, a mixture of 1- and 2-naphthoic acids, remains in the liquid phase, as a molten monoacid. In the closest work of this type, where terephthalic acid and 2,6 - NDA are produced, the oxidation reaction products form as solid crystals in the oxidation reactor, and trap reaction intermediates. Because the naphthoic acid reaction product in the present invention remains in the liquid phase, intermediate aldehydes, and other oxidation intermediates, can be separated, recycled, and reacted to completion, without being entrapped by the product crystals as they form.

### DETAILED DESCRIPTION OF THE DRAWING

The Figure is a process flow diagram illustrating one embodiment of this invention. It is not intended to limit the invention. This process can be one part of a larger integrated process for producing aromatic dicarboxylic acids, as described in copending 60/151,577 (D#TH1432). Those skilled in the art will see many possible variations within the scope of the invention. The liquid phase, catalytic oxidation of methylnaphthalene occurs in reactor **1**. Methylnaphthalene enters the reactor in feed line **4**, fresh oxidation catalyst enters at **6**, acetic acid makeup enters at **5**, recycled solvent from **16** enters at **18** and, along with the catalyst and makeup solvent, is added in line **3**. In practice, the streams represented by stream **3** may enter the reactor individually. A compressed oxygen-containing gas for the oxidation reaction is supplied at **2**. The primary products of the oxidation reaction are naphthoic acid and water. Both isomers of naphthoic acid are produced, 1-naphthoic acid and 2-naphthoic acid, the relative ratio being determined approximately by the relative ratio of 1-methylnaphthalene to 2-methylnaphthalene in the oxidation feed. Minor amounts of trimellitic acid, phthalic acid, other organic compounds, and carbon dioxide are also formed in the reactor, and some methylnaphthalene remains unreacted. The reactor effluent exits the oxidation reactor **1** at **7** and enters a distillation column **8** which separates acetic acid and water off the top, represented by **9**, and recovers the crude naphthoic acid oxidation product which exits the bottom at **10**. The crude naphthoic acid oxidation product **10** enters a steam stripping column **13** where steam is supplied at **14**. Additional acetic acid and water are taken off the top of 13, and combined with stream **9** to create stream **11**. Stream 11 is routed to a distillation column **12**, where waste water exits the top at **15**, and recovered acetic acid exits the bottom at **16** and is recycled to the combined oxidation feed stream **3**.

The crude naphthoic acid, as a liquid, exits column **13** at **17**, at a temperature above its melting point which is about 150°C. The crude naphthoic acid product, **17**, then flows countercurrently to an aqueous wash stream through a first aqueous extraction **19**, a hydrodebromination step **24**, and a second aqueous extraction step **26**. The crude naphthoic acid product first enters aqueous extraction step, **19**. This extraction step effectively removes inorganic bromine, by-product phthalic acid, trimellitic acid, and much of the cobalt and manganese from the oxidation catalyst, all of which exit the wash at **20**, and are recycled to a means for catalyst recovery and regeneration, represented by **21**, which is prior to the point for reentry of the recycled acetic acid, **18**. The water used in the first aqueous extraction step 19, is recycled water which has been cascaded into 19 from the second aqueous extraction 26. After the aqueous extraction in 19, the crude naphthoic acid product is directed, along with added hydrogen **23**, to a hydrodebromination reactor **24** where the crude product is debrominated in the absence of solvent, over a Pd on carbon catalyst to eliminate any brominated organic products formed in the oxidation. The absence of solvent and moderate temperatures distinguishes the debromination from what is known about debromination in the art. Exiting crude naphthoic acid product 25 then enters a second aqueous extraction 26 where it is washed with water to remove residual inorganic bromine compounds created during the hydrodebromination reaction. Fresh water is added at **27**. This is the water that is cascaded back through several steps to effect a countercurrent water wash. At this point in the process, the levels of bromine should be reduced to less than 50 ppm. The washed napthoic acid in this process is then directed toward a salt formation reactor, as part of a larger integrated process.

Following hydrodebromination, the crude naphthoic acid is again extracted with water **26** to remove ionic bromine created during the hydrodebromination reaction. The exiting water **28** is routed to the previous water extraction step.

The hydrodebrominated crude naphthoic acid **29** is contacted in neutralization reactor **30** with a recycled aqueous solution **34** containing potassium bicarbonate. The potassium bicarbonate reacts with naphthoic acid, converting it to potassium naphthoate and extracting it from the organic phase into the aqueous phase as potassium naphthoate. Water and carbon dioxide are formed as by-products, and the carbon dioxide is vented in **37** in order to drive the neutralization reaction to completion. The recycled stream **34** also contains some potassium naphthoate, dipotassium salt of 2,3 - NDA, and low levels of the potassium salt of 2,6 - NDA. The molar ratio of basic potassium to naphthoic acid entering the neutralization reactor ranges from 1 to 1.5, with additional make-up potassium base added in stream **31**. The reactor is run with reflux of water to remove the heat of neutralization of naphthoic acid and aid in the removal of carbon dioxide. A suitable temperature for this neutralization step is in the range of 50 to 200°C, preferably 100 to 150°C.

The present invention will be more clearly understood from the following examples. It is understood that these examples are presented only to illustrate some embodiments of the invention and are not intended to limit the scope thereof.

### EXAMPLE 1

### (Hydrodebromination)

For this example, reactor effluent obtained from continuous oxidation reaction was subjected to rotary vacuum evaporation at 150°C and 6,650 Pa (50 mm Hg) pressure to remove acetic acid and water. The remaining solid material was melted and extracted with an equal weight of water at 150°C. Ten grams of the organic layer, containing approximately 0.7%wt organically bound bromine and 0.2%wt ionic bromine, was charged to a stirred 316 stainless steel autoclave, along with 1 gram of 1%wt palladium/carbon catalyst powder. The gas space of the autoclave was purged with hydrogen, and the pressure was raised to 689 kPag (100 psig) with hydrogen. The autoclave was heated rapidly to 100°C and held at that temperature for at least 10 minutes to assure activation of the catalyst. The autoclave was heated further to 220°C and held at that temperature for 2 hours. The autoclave was allowed to cool to about room temperature, at which time the excess pressure was vented.

The entire contents of the autoclave was removed and mixed with sufficient aqueous potassium hydroxide to dissolve all of the naphthoic acid and Br-naphthoic acid as the corresponding potassium salts, and toluene to dissolve the remaining organic material, including methylnaphthalene and naphthaldehyde. This mixture was filtered to remove the palladium/carbon catalyst, then separated into organic and aqueous layers. The aqueous layer was acidified with aqueous sulfuric acid, to precipitate the naphthoic acid and Br-naphthoic acid. The precipitate was recovered by filtration, rinsed with water, and vacuum-dried at 100°C. This material was found to contain 34%wt 1-naphthoic acid and 67%wt 2-naphthoic acid by HPLC analysis.

In order to maximize removal of ionic bromine trapped in the precipitate, the material was again neutralized with aqueous potassium hydroxide, acidified with aqueous sulfuric acid, filtered, rinsed with water, and dried. This material was found to contain 24 ppmw bromine by X-ray fluorescence analysis.

### EXAMPLE 2

### (Hydrodebromination)

After treatment of the reaction effluent in the same fashion as described in Example 1, 10 grams of the produced organic layer was charged to a stirred 318 stainless steel autoclave along with 1 gram of 0.8% wt palladium/carbon extrudate. After purging and pressuring the autoclave to 689 kPag (100 psig) with hydrogen, the catalyst was activated at 100° C for at least 10 mins. The autoclave was heated further to 280° C and held at that temperature for 2 hrs. Work up of the reaction product in a manner analogous to Example 1. yielded a mixture of 1- and 2-naphthoic acid containing 7 ppmw Br, as measured by x-ray fluorescence analysis.

### EXAMPLE 3

### (Hydrodebromination)

In a manner analogous to Example 2 , 10 grams of a produced organic layer was charged to the autoclave with 1 gram of 0.8% wt palladium/carbon catalyst extrudate. The mixture was activated as before and heated to 260° C for 0.5 hours. The resulting Bromine content after work up was 28 ppmw as measured by x-ray fluorescence analysis.

## Claims

1. A process for hydrodebromination of a molten crude product aromatic carboxylic acid prepared by liquid phase oxidation of an aromatic compound having at least one oxidizable alkyl or acyl group, in the presence of a heavy metal oxidation catalyst, thereby forming an oxidation reaction product mixture containing said crude product aromatic carboxylic acid, said hydrodebromination process comprising:
recovering the crude product aromatic carboxylic acid mixture;
washing the crude product aromatic carboxylic acid mixture with water; and
treating the aromatic carboxylic acid mixture with hydrogen over a catalyst selected from Group VIII of the Periodic Table, optionally on a support, thereby producing aromatic carboxylic acids having significantly reduced bromine content.

2. The process of Claim 1 wherein said crude mixture is a monoacid.

3. The process of Claim 1 wherein the crude product aromatic carboxylic acid is recovered after the oxidation by distillation.

4. The process of Claim 1 wherein said washing of the aromatic carboxylic acid removes inorganic bromine, by-product phthalic acid, trimellitic acid and cobalt and manganese.

5. The process of Claim 1 wherein said debromination takes place at a temperature from 150-320°C.

6. The process of Claim 1 wherein said debromination takes place at a pressure of from 138-2,068 kPag (20-300 psig).

7. The process of Claim 1 wherein said aromatic compound having at least one oxidizable alkyl group is methyl naphthalene and said aromatic carboxylic acid is naphthoic acid.

8. The process of Claim 1 wherein the hydrodebromination catalyst is selected from Group VIII of the Periodic Table.

9. The process of Claim 8 wherein the hydrodebromination catalyst is on a support.

10. The process of Claim 9 wherein said support is selected from the group consisting of alumina, titania and carbon.

11. The process of Claim 10 wherein said catalyst is palladium on carbon.

12. The process of Claim 1 wherein the temperature of the hydrodebromination is in the range of 200 to 300°C.

13. The process of Claim 1 wherein the temperature is in the range of 220 to 280°C.

14. The process of Claim 1 wherein the pressure is in the range of 138 to 2,068 kPag (20 to 300 psig).

15. The process of Claim 14 wherein the pressure is in the range of 345 to 1,380 kPag (50 to 200 psig).

16. The process of Claim 15 wherein the pressure is in the range of 552 to 872 kPag (80 to 120 psig).

17. The process of Claim 1 wherein said organic bromine species in said crude product after hydrodebromination are less than 30 ppmw.

## Patentansprüche

1. Verfahren zur Hydrodebromierung einer geschmolzenen aromatischen Rohprodukt-Carbonsäure, die durch Flüssigphasenoxidation einer aromatischen Verbindung mit zumindest einer oxidierbaren Alkyl- oder Acylgruppe in Gegenwart eines Schwermetall-Oxidationskatalysators hergestellt wird, wodurch eine Oxidationsreaktionsproduktmischung gebildet wird, welche die aromatische Rohprodukt-Carbonsäure enthält, wobei das Hydrodebromierungsverfahren Folgendes umfasst:
das Gewinnen der aromatischen Rohprodukt-Carbonsäuremischung;
das Waschen der aromatischen Rohprodukt-Carbonsäuremischung mit Wasser; und
das Behandeln der aromatischen Carbonsäuremischung mit Wasserstoff über einem Katalysator, der aus Gruppe VIII des Periodensystems ausgewählt ist, gegebenenfalls auf einem Träger, wodurch aromatische Carbonsäuren mit erheblich reduziertem Bromgehalt hergestellt werden.

2. Verfahren nach Anspruch 1, wobei die Rohmischung eine Monosäure ist.

3. Verfahren nach Anspruch 1, wobei die aromatische Rohprodukt-Carbonsäure nach der Oxidation durch Destillation gewonnen wird.

4. Verfahren nach Anspruch 1, wobei das Waschen der aromatischen Carbonsäure anorganisches Brom, das Nebenprodukt Phthalsäure, Trimellithsäure sowie Cobalt und Mangan entfernt.

5. Verfahren nach Anspruch 1, wobei die Debromierung bei einer Temperatur von 150-320°C stattfindet.

6. Verfahren nach Anspruch 1, wobei die Debromierung bei einem Druck von 138-2.068 kPag (20-300 psig) stattfindet.

7. Verfahren nach Anspruch 1, wobei die aromatische Verbindung mit zumindest einer oxidierbaren Alkylgruppe Methylnaphthalin und die aromatische Carbonsäure Naphthoesäure ist.

8. Verfahren nach Anspruch 1, wobei der Hydrodebromierungskatalysator aus Gruppe VIII des Periodensystems ausgewählt ist.

9. Verfahren nach Anspruch 8, wobei der Hydrodebromierungskatalysator auf einem Träger ist.

10. Verfahren nach Anspruch 9, wobei der Träger ausgewählt ist aus der Gruppe, bestehend aus Aluminiumoxid, Titandioxid und Kohlenstoff.

11. Verfahren nach Anspruch 10, wobei der Katalysator Palladium auf Kohlenstoff ist.

12. Verfahren nach Anspruch 1, wobei die Temperatur der Hydrodebromierung im Bereich von 200 bis 300°C liegt.

13. Verfahren nach Anspruch 1, wobei die Temperatur im Bereich von 220 bis 280°C liegt.

14. Verfahren nach Anspruch 1, wobei der Druck im Bereich von 138-2.068 kPag (20-300 psig) liegt.

15. Verfahren nach Anspruch 14, wobei der Druck im Bereich von 345-1.380 kPag (50-200 psig) liegt.

16. Verfahren nach Anspruch 15, wobei der Druck im Bereich von 552-872 kPag (80-120 psig) liegt.

17. Verfahren nach Anspruch 1, wobei die organische Bromart nach der Hydrodebromierung im Rohprodukt weniger als 30 ppmw beträgt.

## Revendications

1. Procédé d'hydrodébromuration d'un acide carboxylique aromatique brut fondu préparé par une oxydation en phase liquide d'un composé aromatique ayant au moins un groupe alkyle ou acyle oxydable, en présence d'un catalyseur d'oxydation de métal lourd, formant ainsi un mélange de produit réactionnel d'oxydation contenant ledit acide carboxylique aromatique brut, ledit procédé d'hydrodébromuration comprenant les étapes consistant à :
récupérer le mélange d'acide carboxylique aromatique brut ;
laver le mélange d'acide carboxylique aromatique brut avec de l'eau ; et
traiter le mélange d'acide carboxylique aromatique avec de l'hydrogène sur un catalyseur choisi dans le groupe VIII du tableau périodique des éléments, éventuellement supporté, produisant ainsi des acides carboxyliques aromatiques ayant une teneur en brome considérablement réduite.

2. Procédé selon la revendication 1, dans lequel ledit mélange brut est un monoacide.

3. Procédé selon la revendication 1, dans lequel l'acide carboxylique aromatique brut est récupéré après l'oxydation par distillation.

4. Procédé selon la revendication 1, dans lequel ledit lavage de l'acide carboxylique aromatique élimine le brome inorganique, l'acide phtalique comme sous-produit, l'acide trimétillique, le cobalt et le manganèse.

5. Procédé selon la revendication 1, dans lequel ladite débromuration est réalisée à une température comprise dans la plage allant de 150 à 320 °C.

6. Procédé selon la revendication 1, dans lequel ladite débromuration est réalisée à une pression comprise dans la plage allant de 138 à 2068 kPag (20 à 300 psig).

7. Procédé selon la revendication 1, dans lequel ledit composé aromatique ayant au moins un groupe alkyle oxydable est le naphtalène de méthyle et ledit acide carboxylique aromatique est l'acide naphtoïque.

8. Procédé selon la revendication 1, dans lequel le catalyseur d'hydrodébromuration est choisi dans le groupe VIII du tableau périodique des éléments.

9. Procédé selon la revendication 8, dans lequel le catalyseur d'hydrodébromuration est supporté.

10. Procédé selon la revendication 9, dans lequel le support est choisi dans le groupe comprenant l'alumine, l'oxyde de titane et le charbon.

11. Procédé selon la revendication 10, dans lequel ledit catalyseur est du palladium sur charbon.

12. Procédé selon la revendication 1, dans lequel la température de l'hydrodébromuration est comprise dans la plage allant de 200 à 300 °C.

13. Procédé selon la revendication 1, dans lequel la température est comprise dans la plage allant de 220 à 280 °C.

14. Procédé selon la revendication 1, dans lequel la pression est comprise dans la plage allant de 138 à 2068 kPag (20 à 300 psig).

15. Procédé selon la revendication 14, dans lequel la pression est comprise dans la plage allant de 345 à 1380 kPag (50 à 200 psig).

16. Procédé selon la revendication 15, dans lequel la pression est comprise dans la plage allant de 552 à 872 kPag (80 à 120 psig).

17. Procédé selon la revendication 1, dans lequel la teneur en dit brome organique dans ledit produit brut après l'hydrodébromuration est inférieure à 30 ppm en poids.
